# EUROPÄISCHE PATENTANMELDUNG

(11) **EP 1 236 480 A1**
(43) Veröffentlichungstag der Anmeldung: **04.09.2002**
(21) Anmeldenummer: 02002125.9
(22) Anmeldetag: 29.01.2002
(51) Int. Cl.: A61M 25/01, A61B 17/12

(54) **Ureter-Drainagevorrichtung**

(30) Priorität: 02.03.2001 DE 20103653 U
(71) Anmelder: B. BRAUN MELSUNGEN AG, 34212 Melsungen (DE)
(72) Erfinder: Fuchs, Jürgen, 34308 Bad Emstal (DE); Haacke, Claus, Dr., 34212 Melsungen (DE)
(74) Vertreter: Selting, Günther, Dipl.-Ing.

(57) **Zusammenfassung**

Die Ureter-Drainagevorrichtung weist einen Ureterkatheter (10) auf, der über eine Kupplungsvorrichtung (11) lösbar mit einem Schiebeschlauch (12) verbunden ist. Zum Lösen der Kupplungsvorrichtung (11) dient ein Steuerschlauch (18), der über den Schiebeschlauch geschoben ist. Nach dem Zurückziehen des Steuerschlauchs (18) geht die Kupplungsvorrichtung selbsttätig auf, so dass der Schiebeschlauch (12) und der Steuerschlauch (18) von dem Ureterkatheter (10) abgezogen werden können. Eine Handhabungsvorrichtung (13) weist zwei Griffteile (14, 15) auf, von denen eines mit dem Schiebeschlauch (12) und das andere mit dem Steuerschlauch (18) verbunden ist. Die beiden Griffteile (14, 15) werden von einer Feder (16) auseinandergedrückt.

## Beschreibung

Die Erfindung betrifft eine Ureter-Drainagevorrichtung mit einem Ureterkatheter, einem an das proximale Ende des Ureterkatheters ankuppelbaren Schiebeschlauch und einem über den Schiebeschlauch schiebbaren Steuerschlauch.

Ureterkatheter, die auch als Doppel-J-Stents oder innere Harhleiterschienen bezeichnet werden, sind schlauchförmige Hilfsmittel zur Ableitung des Urins aus dem Nierenbecken in die Harnblase, wenn der Harnleiter nicht mehr ausreichend durchgängig ist. Die heute üblichen Harnleiterkatheter haben an beiden Ende Einrollungen, wobei die Einrollung im Nierenbecken einen kleineren Durchmesser besitzt, während die Einrollung der Blase einen größeren Durchmesser hat. Zur Verlegung der Katheter durch die Harnröhre und die Blase wird ein Cystoskop benutzt. Die Einrollungen des Katheters müssen gerade gestreckt werden, damit er durch die Cystoskopkanüle hindurch passt. Das Strecken das Katheters geschieht mit einem Führungsdraht. Wenn die Katheterspitze eine geschlossene Kuppe hat, wird der Führungsdraht vom blasenseitigen Ende eingestellt. Liegt der Führungsdraht bereits im Harnleiter, wird das Strecken und anschließende Hochschieben des Katheters in das Nierenbecken dadurch ermöglicht, dass die Katheterspitze offen und konisch angeformt ist. Weil der Ureterkatheter zur inneren Schienung dient und sich seine drainierende Endregion nur bis in die Blase erstreckt, muss der im Körper des Patienten verbleibende Ureterkatheter vom Führungsdraht abgeschoben werden. Dies geschieht mit einem Schiebeschlauch.

In gewissen Fällen mit sehr stark gewundenem Harnleiterverlauf oder bei sehr engen Harnleiterstenosen ist eine feste, jedoch lösbare Verbindung zwischen dem Harnleiterkatheter und dem Schiebeschlauch zweckmäßig, weil dann der Katheter nicht nur geschoben sondern auch gezogen und gedreht werden kann.

EP 0 363 581 B1 beschreibt eine Ureter-Drainagevorrichtung mit Schiebeschlauch. Der Schiebeschlauch steht mit dem Ureterkatheter über eine Kupplungsvorrichtung aus zwei ineinander greifenden Kupplungselementen in Verbindung. Die Sicherung der Kupplungsvorrichtung in der Eingriffsstellung erfolgt durch den Führungsdraht. Wird der Führungsdraht herausgezogen, so wird der Schiebeschlauch von dem Ureterschlauch gelöst.

In EP 0 752 251 A1 ist eine Ureter-Drainagevorrichtung beschrieben, bei der das proximale Ende des Ureterkatheters über einen Reibungssitz mit einem Schiebeschlauch verbunden ist. In dem Schiebeschlauch erstreckt sich ein Steuerschlauch, der vorgeschoben werden kann, um den Reibungssitz zu lösen und den Ureterkatheter vom Schiebeschlauch zu trennen.

Der Erfindung liegt die Aufgabe zugrunde, eine Ureter-Drainagevorrichtung zu schaffen, bei der der Ureterkatheter sowohl beim Vorschieben als auch beim Zurückziehen sicher an dem Schiebeschlauch festgehalten wird, unabhängig davon, ob ein Führungsdraht eingesetzt ist.

Die Lösung dieser Aufgabe erfolgt erfindungsgemäß mit den im Anspruch 1 angegebenen Merkmalen. Hiernach weisen der Ureterkatheter und der Schiebeschlauch zusammengreifende Kupplungselemente auf, wobei mindestens ein Kupplungselement des Schiebeschlauchs radial nach außen in Löserichtung vorgespannt ist und von dem überdeckenden Steuerschlauch in Eingriffsstellung gehalten wird.

Die Erfindung ermöglicht die Verwendbarkeit der bisher üblichen Katheter-Verlegetechniken, wie die Seldinger-Technik und die retrograde Technik. Sie ermöglicht das leichte und mit definiertem Kraftaufwand durchführbare Abkoppeln des Ureterkatheters von dem Schiebeschlauch. Dies geschieht durch zwei formschlüssig zusammengreifende Kupplungsteile, die von dem äußeren Steuerschlauch in Eingriff gehalten werden. Ein innenliegender Führungsdraht ist bei dieser Anordnung zum Fixieren der beiden Teile nicht nötig. Mindestens eines der Kupplungselemente des Schiebeschlauchs ist radial nach außen in Löserichtung vorgespannt und wird von dem Schiebeschlauch niedergehalten, so dass es in Eingriff mit dem proximalen Ende des Ureterkatheters bleibt. Auf diese Weise können sowohl Zug- als auch Schiebe- und Drehkräfte übertragen werden. Zum Lösen der Verbindung wird der Steuerschlauch auf dem Schiebeschlauch zurückgezogen, wobei das Kupplungselement sich unter Wirkung der Vorspannung auseinander spreizt und den Ureterkatheter freigibt.

Gemäß einer bevorzugten Ausgestaltung der Erfindung ist es vorgesehen, dass das Kupplungselement des Schiebeschlauchs mindestens ein distal abstehendes Bein mit einem endseitig vorgesehenen Haken aufweist, und dass das Kupplungselement des Ureterkatheters eine Ausnehmung zur Aufnahme des Hakens aufweist.

Vorzugsweise ist das Kupplungselement des Schiebeschlauchs als Widerhaken ausgebildet, der beim Aufschieben in eine Ausnehmung des Ureterkatheters einrastet. Dieser Widerhaken hat eine Sägezahnform mit einer Schrägflanke und einer Steilflanke.

Bei einer bevorzugten Ausführungsform der Erfindung ist eine Handhabungsvorrichtung vorgesehen, welche zwei in Längsrichtung verschiebbare und auseinandergedrückte Griffteile aufweist, von denen eines mit dem Schiebeschlauch und das andere mit dem Steuerschlauch verbunden ist. Die Griffteile werden von einer Federvorrichtung auseinandergedrückt, wobei der Steuerschlauch in die Vorschubstellung getrieben wird, in der er die Kupplungsvorrichtung überdeckt und verriegelt. Wird das mit dem Steuerschlauch verbundene Griffteil entgegen der Federkraft zurückgezogen, so gibt der Steuerschlauch die Kupplungsvorrichtung zum Lösen frei. Dadurch ist eine einfache Handhabung und sogar eine Einhandbedienung der Drainagevorrichtung zum Lösen der Kupplungsvorrichtung möglich.

Im Folgenden wird ein Ausführungsbeispiel der Erfindung unter Bezugnahme auf die Zeichnungen näher erläutert.

Es zeigen:
- Fig. 1: einen Längsschnitt durch die Drainagevorrichtung in dem Zustand, in dem der Ureterkatheter in den Körper eingebracht wird,
- Fig. 2: eine vergrößerte Darstellung der Einzelheit II aus Fig. 1,
- Fig. 3: die Vorrichtung mit eingesetztem Führungsdraht,
- Fig. 4: die Vorrichtung in dem Zustand, in dem der Ureterkatheter von dem Schiebeschlauch gelöst wird, und
- Fig. 5: eine perspektivische schematische Darstellung der Kupplungsvorrichtung.

Die dargestellte Ureter-Drainagevorrichtung weist einen Ureterkatheter 10 auf, der aus einem flexiblen Schlauch besteht und im entspannten Zustand an jedem der beiden Enden eine Einrollung aufweist. In den Zeichnungen ist der Ureterkatheter 10 in gestrecktem Zustand und ohne nierenseitige Einrollung dargestellt.

Das proximale Ende 10a des Ureterkatheters 10 ist über eine Kupplungsvorrichtung 11 mit einem Schiebeschlauch 12 verbunden, welcher den Ureterkatheter verlängert. Das proximale Ende des Schiebeschlauchs 12 ist mit einem ersten Griffteil 14 und einer Handhabungsvorrichtung 13 verbunden. Auf dem ersten Griffteil 14 ist ein zweites Griffteil 15 in Längsrichtung verschiebbar. Eine Feder 16 drückt die beiden Griffteile 14 und 15 in axialer Richtung auseinander, wobei ein Anschlag 17 die Bewegung des zweiten Griffteils 15 begrenzt.

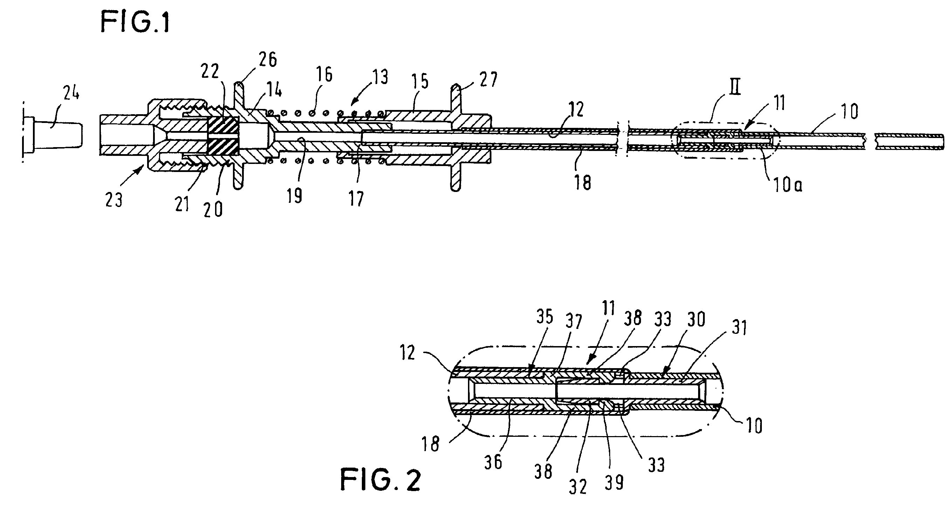

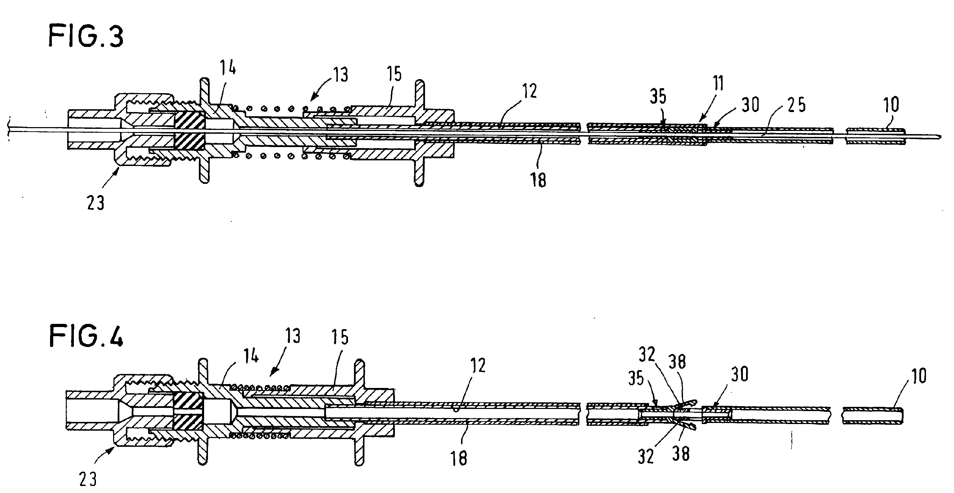

Das zweite Griffteil 15 ist mit einem Steuerschlauch 18 verbunden, welcher den Schiebeschlauch 12 umgibt. Durch Bewegen des zweiten Griffteils 15 relativ zum ersten Griffteil 14 kann der Steuerschlauch 18 auf dem Schiebeschlauch verschoben werden. Das erste Griffteil 14 enthält einen längslaufenden Kanal 19, der sich an das Lumen des Schiebeschlauchs 12 anschließt und mit einem Anschlusselement 20 des Griffteils 14 in Verbindung steht. Das Anschlusselement 20 weist ein Außengewinde 21 auf und enthält einen ringförmigen Elastomerring 22. Auf das Anschlusselement 20 kann ein Verbindungsstück 23 aufgeschraubt werden, welches das Setzen einer Spritze 24 (Fig. 1) oder das Einführen eines Führungsdrahts 25 (Fig. 3) ermöglicht.

Das erste Griffteil 14 weist einen radial abstehenden Griff 26 auf und das zweite Griffteil 15 weist ebenfalls einen radial abstehenden Griff 27 auf. Durch Heranziehen des Griffs 27 an den Griff 26 kann der Steuerschlauch 18 auf dem Schiebeschlauch 12 zurückgezogen werden.

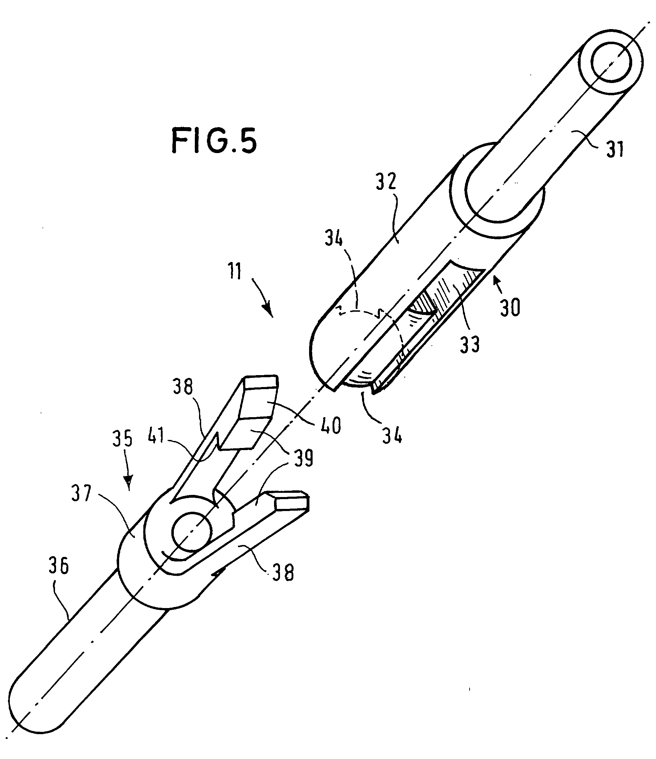

Fig. 2 zeigt in vergrößertem Maßstab die Kupplungsvorrichtung 11. Diese weist ein erstes Kupplungsstück 30 auf, das mit einem Rohransatz 31 in den Ureterkatheter 10 hineinragt und dort fixiert ist. An den Rohransatz 31 schließt sich ein Kopfstück 32 größeren Durchmessers an, das zwei diametral gegenüberliegende Ausnehmungen 33 aufweist. In diese Ausnehmungen 33 führen gemäß Fig. 5 längslaufende Führungsnuten 34 hinein.

In das distale Ende des Schiebeschlauchs 12 ist ein Kupplungsstück 35 eingesetzt, das einen Rohransatz 36 und ein außerhalb des Schieberohrs befindliches Kopfstück 37 aufweist. Von dem Kopfstück 37 erstrecken sich zwei Beine 38 mit jeweils einem endseitig vorgesehenen Haken 39. Die Haken 39 haben jeweils eine Schrägflanke 40 und eine Steilflanke 41. Sie sind nach außen vorgespannt, d. h. sie sind bestrebt sich gemäß Fig. 5 auseinander zu spreizen. Bei ineinander gesteckten Kupplungsteilen 30, 35 gemäß Fig. 2 dringen die Haken 39 in die Ausnehmungen 33 ein, wenn von dem Steuerschlauch 18 eine die Beine 38 nach Innen drückende Kraft ausgeübt wird. Wird der Steuerschlauch 18 dagegen zurückgezogen, so spreizen sich die Beine 38 gemäß Fig. 5 ab und die Haken 39 verlassen die Ausnehmungen 33. Die Kupplungsvorrichtung 11 ist daher so ausgebildet, dass sie in Löserichtung vorgespannt ist.

Fig. 1 zeigt den Ureterkatheter 10 nach dem Ankoppeln an den Schiebeschlauch 12 mit Hilfe der Kupplungsvorrichtung 11. Hierbei ist der Steuerschlauch 18 bereits vorgeschoben, so dass er die federnden Beine 38 überdeckt und im verriegelten Zustand hält.

Gemäß Fig. 3 wird der Führungsdraht 25 durch das Verbindungsstück 23 und durch die Handhabungsvorrichtung 13 hindurch in den Schiebeschlauch 12 und den Ureterkatheter 10 eingeschoben, der dadurch in eine Geradeausrichtung gestreckt wird. Der Führungsdraht kann dann in der vorgesehenen Position in der Handhabungsvorrichtung fixiert werden, indem das Verbindungsstück 23 festgeschraubt wird. Dadurch wird der Elastomerring 22 axial zusammengequetscht, so dass sich seine Öffnung verringert und der Führungsdraht 25 im Elastomerring 22 eingeklemmt wird. Das Verbindungsstück 23 wirkt daher auch als Spannvorrichtung zum Klemmen des Führungsdrahtes.

In dem in Fig. 3 dargestellten Zustand wird die Vorrichtung durch ein Cystoskop in den Körper des Patienten eingeführt, wobei der Ureterkatheter 10 in dem Harnleiter platziert wird. Dann wird gemäß Fig. 4 die Handhabungsvorrichtung 13 betätigt, indem das zweite Griffteil 15 an das erste Griffteil 14 herangezogen wird, wobei die Feder 16 gespannt wird. Hierdurch wird der Steuerschlauch 18 zurückgezogen, so dass er die Kupplungsvorrichtung 11 freigibt und die Beine 38 sich selbsttätig nach außen spreizen. Nun kann die Handhabungsvorrichtung 13 zusammen mit dem Schiebeschlauch und dem Steuerschlauch 18 zurückgezogen werden, wobei der Ureterkatheter 10 im Harnleiter verbleibt.

Falls die Katheterverlegung ohne Führungsdraht 25 erfolgt, kann mit der Spritze 24 ein Röntgenkontrastmittel in den Schiebeschlauch 12 und den Ureterkatheter 10 eingegeben werden, so dass die Katheterplatzierung im Röntgenbild kontrolliert werden kann.

Alternativ zu der nachträglichen Einbringung des Führungsdrahts 25 besteht auch die Möglichkeit, zuerst den Führungsdraht in den Patientenkörper einzubringen und dann die beschriebene Vorrichtung über den Führungsdraht zu schieben.

## Patentansprüche

1. Ureter-Drainagevorrichtung mit einem Ureterkatheter (10), einem an das proximale Ende des Ureterkatheters ankoppelbaren Schiebeschlauch (12) und einem über den Schiebeschlauch schiebbaren Steuerschlauch (18),
**dadurch gekennzeichnet,**
**dass** der Ureterkatheter (10) und der Schiebeschlauch (12) zusammengreifende Kupplungselemente (33, 38) aufweisen, wobei mindestens ein Kupplungselement (38) des Schiebeschlauchs (12) radial nach außen in Löserichtung vorgespannt ist und von dem überdeckenden Steuerschlauch (18) in Eingriffsstellung gehalten wird.

2. Ureter-Drainagevorrichtung nach Anspruch 1, **dadurch gekennzeichnet, dass** das Kupplungselement (38) des Schiebeschlauchs mindestens ein distal abstehendes Bein mit einem endseitig vorgesehenen Haken (39) aufweist und dass das Kupplungselement (33) des Ureterkatheters (10) eine Ausnehmung zur Aufnahme des Hakens (39) aufweist.

3. Ureter-Drainagevorrichtung nach Anspruch 1 oder 2, **dadurch gekennzeichnet, dass** das proximale Ende (10a) des Ureterkatheters (10) ein eingesetztes Kupplungsstück (30) aufweist.

4. Ureter-Drainagevorrichtung nach einem der Ansprüche 1 - 3, **dadurch gekennzeichnet, dass** das Kupplungselement (38) des Schiebeschlauchs (12) als Widerhaken ausgebildet ist, der beim Aufschieben in einer Ausnehmung (33) des Ureterkatheters (10) einrastet.

5. Ureter-Drainagevorrichtung nach einem der Ansprüche 1 - 4, **gekennzeichnet durch** eine Handhabungsvorrichtung (13), welche zwei in Längsrichtung verschiebbare und auseinandergedrückte Griffteile (14, 15) aufweist, von denen eines mit dem Schiebeschlauch (12) und das andere mit dem Steuerschlauch (18) verbunden ist.

6. Ureter-Drainagevorrichtung nach Anspruch 5, **dadurch gekennzeichnet, dass** die Griffteile (14, 15) seitlich abstehende Griffe (26, 27) aufweisen.
